# EUROPÄISCHE PATENTSCHRIFT

(11) **EP 3 203 897 B1**
(45) Veröffentlichungstag und Bekanntmachung des Hinweises auf die Patenterteilung: **20.03.2019**
(21) Anmeldenummer: 16751282.1
(22) Anmeldetag: 09.08.2016
(51) Int. Cl.: A61B 1/005, G02B 23/24, A61B 1/00

(54) **HANDGRIFF EINES ENDOSKOPS**
HANDLE FOR AN ENDOSCOPE
POIGNÉE D'UN ENDOSCOPE

(30) Priorität: 14.08.2015 DE 102015113426
(43) Veröffentlichungstag der Anmeldung: 16.08.2017
(73) Patentinhaber: Ambu A/S, 2750 Ballerup (DK)
(72) Erfinder: FIEBER, Markus, 80636 München (DE); ABICHT, Felix, 82337 Walchstadt (DE); SCHÜTZ, Günter, 86152 Augsburg (DE); ILG, Dylan, 82266 Inning (DE)
(74) Vertreter: Winter, Brandl, Fürniss, Hübner, Röss, Kaiser, Polte - Partnerschaft mbB
(86) Internationale Anmeldenummer: PCT/EP2016/068953
(87) Internationale Veröffentlichungsnummer: WO 2017/029157

(56) Entgegenhaltungen:
- JP-A- H0 815 614
- US-A- 5 873 814
- US-A1- 2002 095 088
- US-A1- 2002 103 418
- US-A1- 2008 064 925
- US-A1- 2008 200 758
- US-A1- 2011 009 694
- US-A1- 2011 251 459
- US-A1- 2014 275 763
- US-A1- 2014 288 460

## Beschreibung

Die vorliegende Erfindung betrifft den als separates, adaptives Bauteil ausgebildeten Handgriff eines Endoskops (einschließlich Koloskop und/oder Gastroskop) mit Optik, Beleuchtung sowie einem Arbeitskanal mit Endoskop-spezifischer Funktion und Dimensionierung gemäß dem Oberbegriff des Patentanspruchs 1.

### Hintergrund der Offenbarung

Endoskope sind medizinische Arbeitsgerätschaften, mittels denen das Innere von in der Regel lebenden Organismen, aber auch technischen Hohlräumen untersucht und/oder manipuliert werden kann. Ursprünglich für die humanmedizinische Diagnostik entwickelt, werden sie heute auch für minimal-invasive operative Eingriffe an Mensch und Tier sowie in der Industrie zur Sichtprüfung schwer zugänglicher Hohlräume eingesetzt. Zu den Endoskopen zählen die starren und flexiblen Endoskope und deren Unterarten.

Ein starres Endoskop leitet die Bildinformationen des zu untersuchenden Objekts bzw. Raums durch ein Linsensystem im Inneren des Endoskopschafts bzw. im distalen Endoskopkopf an ein proximal angeordnetes Okular weiter. Beispiele hierfür sind das technische Boreskop und med. das Arthroskop und Zystoskop.

Das für die Untersuchung/Inspektion notwendige Licht wird entweder durch ein distales Leuchtmittel (z.B. LED) im Endoskopkopf bereitgestellt oder über einen an ein proximales Leuchtmittel angeschlossenen Lichtleiter, ebenfalls im Inneren des Schafts durch Glasfaserbündel an die Spitze des Endoskops transportiert.

Bei einem flexiblen Endoskop werden Bild und Licht ebenfalls häufig über Glasfaserbündel übertragen. Beispiele sind das technische Flexoskop und das med. Gastroskop, Koloskop und Bronchoskop.

Ab einem praktikablen Durchmesser sind flexible (und auch starre) Endoskope auch mit auswechselbaren statt festmontierten Objektiven (*Vor*/*Seit* oder *Rückwärts*) sowie zusätzlichen Arbeitskanälen zum Einführen von mikromechanischen Geräten (kleine Zangen oder Greifer) in den Untersuchungs- bzw. Inspektionsraum erhältlich. Flexible Endoskope besitzen meist eine über eingebaute Bowdenzüge oder Hydraulik-Stellantriebe fernsteuerbare Gerätespitze (Endoskopkopf). Diese kann je nach Modell und Durchmesser nach 2 (*auf-ab*) oder nach 4 Seiten (*auf-ab* und *rechts-links*) teilweise bis zu 180° abgewinkelt werden. Die Länge dieser abwinkelbaren Spitze kann je nach Durchmesser zwischen 30 und 70 mm liegen. Im proximalen Handgriff des Geräts/Endoskops ist eine Mechanik eingebaut, die über Kippbügel oder Handräder auf die Bowdenzüge/Stellantriebe einwirkt und die Bewegung der Spitze ermöglicht.

Die jüngste Unterart der flexiblen Endoskope bilden die Videoendoskope, oft auch Videoskope genannt. Videoendoskope eröffnen ein neues Kapitel in der modernen Endoskopie, da sie zur Bilderzeugung und -übertragung digitale Technologien nutzen. Ein am distalen Objektiv des Videoendoskops angebrachter CCD- bzw. CMOS-Chip erzeugt ein digitales Bild des Untersuchungsobjekts und leitet es an die folgenden Baugruppen des Videoendoskops weiter. Meist bereitet dann ein Prozessor diese Daten auf, sendet sie zur Ausgabe an einen Monitor, oder legt sie auf einer Festplatte oder anderen Datenspeichern ab. Videoendoskope ermöglichen je nach Ausstattungsvariante das Einfrieren und Speichern von Bildern sowie mehrstündige Videoaufzeichnungen für eine nachträgliche Aufbereitung/Optimierung oder auch das Vermessen eines Bildes bzw. Objektes. Auch diese Gerätetechnik besitzt eine fernsteuerbar, abwinkelbare Gerätespitze, nach 2 oder 4 Richtungen. Diese können mechanisch oder elektronisch/hydraulisch gesteuert werden. Die mechanische Steuerung erfolgt über ein kleines Getriebe über Kipphebel oder Drehräder. Einige Videoskope haben anstelle der Mechanik kleine Elektromotoren oder Hydraulikbalge eingebaut, die beispielsweise über einen Joystick ansteuerbar sind.

Neuerdings kommen bei Videoskopen anstelle der externen Lichtprojektoren, auch LED-Leuchtkörper zum Einsatz. Diese können in der Gerätespitze eingebaut sein, oder auch im proximalen Handgriff. Im letzteren Fall wird dann das Licht über eingebaute Glasfasern nach vorn zur Spitze geleitet.

Zusammenfassend sind demnach starre wie auch flexible Endoskope der einschlägigen Gattung (einschließlich des vorliegenden Offenabrungsgegenstands) gekennzeichnet durch einen Endoskopschaft (flexibel oder starr), an dessen distalem Ende (Endoskopkopf) eine Beleuchtung (Lichtaustritt eines Lichtleiterkabels oder ein Leuchtkörper) sowie eine Optik (Linsensystem und/oder lichtempfindlicher Chip) angeordnet sind. Innerhalb des Endoskopschafts ist (neben optionalen diversen Funktions-/Versorgungskanälen beispielsweise für die Optik, die Beleuchtung und/oder die Abkrümmeinrichtung der Instrumentenspitze) ein Arbeitskanal ausgeformt, der dafür vorgesehen und angepasst ist, chirurgische Instrumente durch das Endoskop hindurch in den Patientenkörper einzuführen, um unter visueller Kontrolle über die Optik und die Beleuchtung (minimalinvasive) Operationen/Behandlungen vorzunehmen.

Da chirurgische Instrumente für minimalinvasive Eingriffe eine Mindestgröße aufweisen, hat der Arbeitskanal einen entsprechenden Durchmesser, der das Einführen der chirurgischen Instrumente erlaubt. Dieser Durchmesser ist in der Regel deutlich größer als der der Funktions-/Versorgungskanäle.

Neben der vorstehend beschriebenen Funktion hat der Arbeitskanal aber auch die Aufgabe, beispielsweise Behandlungsflüssigkeiten (Medikamentlösungen, etc.) zu fördern oder Patientengewebe und/oder Körperflüssigkeiten abzutransportieren. Außerdem kann über den Arbeitskanal Spülflüssigkeit an die Behandlungsstelle im Patientenkörper gepumpt werden.

### Stand der Technik

Aus dem Stand der Technik sind allgemein Endoskope der vorstehenden Gattung (starre und flexible Endoskope) bekannt, bei denen der Arbeitskanal sowohl an der distalen Endoskopspitze wie auch im Bereich des proximalen Handgriffs jeweils eine Austrittsöffnung hat. Über die proximale Öffnung kann dann beispielsweise ein chirurgisches Instrument eingeführt werden oder es kann eine Druck-/Saugpumpe angeschlossen werden.

Das Problem dieser Endoskope besteht jedoch darin, dass insbesondere bei minimalinvasiven chirurgischen Eingriffen bei in den Arbeitskanal eingeführtem chirurgischem Instrument gleichzeitig beispielsweise Spüllösung an die Operationsstelle herangeführt werden muss. Dies erfolgt dann über einen zum Arbeitskanal separat im Endoskopschaft ausgebildeten Versorgungskanal mit vergleichsweise kleinem Durchmesser. Es hat sich hier aber gezeigt, dass nur wenig Spüllösung über diesen engen Versorgungskanal pro Zeiteinheit gefördert werden kann, was sich insbesondere bei besonders langen Endoskopschäften (bis zu 250cm Länge) insoweit negativ auswirkt, als dass die Spüllösung mit hohem Druck in den Versorgungskanal gepumpt werden muss, um dieses in ausreichender Menge pro Zeiteinheit bis zur distalen Endoskopspitze fördern zu können.

Die Dokumente US 2014 / 0275763 A1, US 2008 / 0200758 A1, US 2002 / 0103418 A1 offenbaren jeweils ein Endoskop mit einem Handgriff. Weiterer gattungsgemäßer Stand der Technik ist aus den Dokumenten US 2011 / 0251459 A1, JP H08 15614 A, US 2008 / 064925 A1 und US 2014 / 288460 A1 bekannt.

Weiterhin offenbart die US 2011 / 0009694 A1 einen Handgriff eines Endoskops über den eine Hülle stülpbar ist. Die US 5,873,814 offenbart ein Endoskopgriffmonitor, der mit einer resistenten Folie beschichtet ist. Letztlich offenbart die US 2002/0095088 A1 einen Handgriff eines Endoskops, auf den eine Folie auf einem Bedienfeld angeordnet ist.

### Kurzbeschreibung der Offenbarung

Angesichts dieser, von der vorliegenden Anmelderin erkannten Problematik ist es die Aufgabe der Offenbarung, ein Endoskop dieser Gattung (vorzugsweise flexibel aber ggf. auch starr) bereitzustellen, mittels dem eine ausreichende Flüssigkeitsver- und/oder - entsorgung auch bei gleichzeitig in den Arbeitskanal eingeführtem chirurgischen Instrument möglich ist.

Ferner soll vorzugsweise die Handhabung des Endoskops verbessert werden, indem auf einfache Weise Teile des Endoskops als Ein-Weg-Artikel nach Gebrauch einfach entsorgt und andere Teile des Endoskops als Mehr-Weg-Artikel nach Gebrauch auf einfache Weise gereinigt werden können. Schließlich soll weiter vorzugsweise der Betrieb des Endoskops verbessert werden.

Diese Aufgabe wird erfindungsgemäß durch ein Endoskop mit den Merkmalen des Anspruchs 1 gelöst. Vorteilhafte Ausgestaltungen der Erfindung sind Gegenstand der Unteransprüche.

Ein Kerngedanke der Offenbarung ist es, den Arbeitskanal zum Zwecke des Einführens von chirurgischen Instrumenten aber auch des Zu- und/oder Abführens von Fluiden/Flüssigkeiten besser zu nutzen. Die gegenüber dem Stand der Technik verbesserte Ausnutzung des Arbeitskanals wird dadurch erreicht, indem dem Arbeitskanal mindestens zwei proximale Anschlüsse zugeordnet sind, welche separat und unabhängig voneinander (damit auch parallel/gleichzeitig) genutzt werden können, um jeweils ein Medium (ein chirurgisches Instrument und/oder ein Fluid) über jeden proximalen Anschluss in den Arbeitskanal (parallel/gleichzeitig) ein- und/oder abzuführen.

In anderen Worten ausgedrückt hat der (einzige) Arbeitskanal des Endoskops wenigstens zwei proximale Anschlüsse, von denen wenigstens ein proximaler Anschluss für das Einführen eines (medizinischen) Gegenstands wie chirurgisches Instrument geeignet und vorzugsweise angepasst ist und wenigstens ein anderer proximaler Anschluss für das Einführen/Entnehmen eines Fluids wie eine Flüssigkeit (z.B. Spüllösung, Körperflüssigkeit, etc.) und/oder ein Gas (z.B. Luft zum Entfalten eines Patientendarms) geeignet und vorzugsweise angepasst ist.

Auf diese Weise kann der Arbeitskanal auch bei eingeschobenem/eingeführtem chirurgischen Instrument gleichzeitig zur Zufuhr und/oder Entnahme von Fluid genutzt werden. Da folglich ein im Stand der Technik hierfür gesondert vorgesehener Ver- / Entsorgungskanal entfallen kann, lässt sich der Arbeitskanal vergleichsweise großzügig dimensionieren, ohne dass der Endoskopschaft-Außendurchmesser hierfür aufgeweitet werden müsste.

Die Anpassung des einen Anschlusses für das Einführen eines chirurgischen Instruments definiert sich im einfachsten Fall im Anschluss-Inndurchmesser, der so groß sein muss, dass ein chirurgisches Instrument hineingeschoben werden kann. In anderen Worten ausgedrückt orientiert sich der Anschluss-Durchmesser am Durchmesser des Arbeitskanals. Außerdem sollte dieser Anschluss vorzugsweise einen sanften Eingang zum Arbeitskanal aufweisen, also etwa einen inneren Bogen ausführen oder axial zum Arbeitskanal verlaufen, damit das einzuführende chirurgische Instrument nicht abgeknickt wird.

Die Anpassung des Anschlusses für das Ein-/Ausführen eines Fluids definiert sich hingegen im einfachsten Fall durch die Ausbildung einer Verschlussmöglichkeit. In anderen Worten ausgedrückt hat dieser Anschluss eine zusätzliche Einrichtung, an welcher beispielsweise ein Schlauch (fluiddicht) angeschlossen werden kann. Z.B. kann diese ein Rohrstutzen/Nippel sein, auf welchem ein Schlauch aufgesteckt werden kann, oder ein Bajonett-, Schraub- oder Lueranschluss.

Vorzugsweise werden die mehreren Anschlüsse des Arbeitskanals durch einen weiter vorzugsweise einstückigen Anschlusseinsatz gebildet. Dieser Anschlusseinsatz hat (besteht) in einem bevorzugten Ausführungsbeispiel einen Rohrlauf, dessen eines Ende mit dem Arbeitskanal (fluid-)verbunden (verbindbar) ist und an dessen anderem Ende ein Anschlussstutzen angeordnet ist mit einer Anzahl von Anschlussrohren, die sämtlich in den Rohrlauf einmünden. Vorzugsweise ist der Anschlusseinsatz fest mit dem Endoskopschaft an dessen proximalem Ende verbunden. Sofern der Endoskopschaft biegeflexibel ist, weist der Anschlusseinsatz deutlich starrere (steifere) Eigenschaften auf.

Vorteilhafter Weise ist der Rohrlauf dafür angepasst, in einen Handgriff eingesetzt zu werden, derart, dass die Anschlussrohre des Anschlussstutzens zumindest abschnittsweise aus dem Handgriff herausragen, bzw. von außen zugänglich sind. Der Handgriff ist somit von den Anschlüssen separiert und wird daher nicht oder nur geringfügig kontaminiert.

Gemäß einem ggf. unabhängig zu beanspruchenden Aspekt der vorliegenden Offenbarung ist der Handgriff als (lösbarer) Aufsteckhandgriff ausgebildet, der vorzugsweise mit dem Anschlusseinsatz und weiter vorzugsweise mit dem Rohrlauf des Anschlusseinsatzes weiter vorzugsweise klemmend zusammenwirkt. D.h. das Endoskop besteht im Wesentlichen aus zwei Baueinheiten, nämlich dem Endoskopschaft (mit dem vorzugsweise flexiblen, in den Patientenkörper einzuführenden Schaftabschnitt mit Endoskopkopf und den darin verbauten Funktionen sowie mit dem vorzugsweise starren Anschlusseinsatz) und dem separat hierzu ausgebildeten Handgriff, der einfach am Endoskopschaft (Anschlusseinsatz) temporär fixiert werden kann.

Hierfür kann der Rohrlauf des Anschlusseinsatzes eine Anzahl von Rastelementen oder Hinterschneidungen haben, die mit entsprechenden Rastelementen oder Hinterschneidungen auf Seiten des Handgriffs zusammenwirken. Vorzugsweise bildet der Handgriff einen Clip-/Schalenabschnitt mit einer Anzahl von Hinterschneidungen und/oder Vorsprüngen, derart, dass der Handgriff auf den Rohrlauf aufgeclipt/aufgeklippt werden kann, wobei entsprechende Vorsprünge und/oder Hinterschneidungen auf Seiten des Clipabschnitts mit den Hinterschneidungen und/oder Vorsprüngen am Rohrlauf in Formschluss kommen. Dadurch wird ein sicherer (aber lösbarer) Halt des Handgriffs auf dem Rohrlauf gewährleistet.

Wie vorstehend bereits angedeutet wurde, ist der Anschlusseinsatz vorzugsweise fest mit dem für das Einführen in den Patientenkörper vorgesehenen (biegeflexiblen) Endoskopschaftabschnitt gekoppelt. Der Endoskopschaft (einschließlich des Anschlusseinsatzes) ist hierbei vorzugsweise als Einweg-Gegenstand konzipiert. Da aber der Handgriff keinen unmittelbaren Kontakt beispielsweise mit Körperflüssigkeit des Patienten bekommt (sämtlich Leitungen und Anschlüsse sind im Endoskopschaft), kann dieser als Mehrweg-Produkt vorgesehen sein. Daher ist die Kopplung zwischen Handgriff und Anschlusseinsatz lösbar, vorliegend vorzugsweise als Clickverbindung gedacht. Es könnte aber jedwede andere lösbare Kopplung wie Einsteck-, Klett, Spann- oder Schraubverbindung vorgesehen sein.

Vorzugsweise sind am Anschlusseinsatz weitere Anschlüsse für die Versorgungs-/Funktionskanäle und/oder die Funktionen wie Optik, Beleuchtung, Stellantriebe, etc. ausgebildet. Auf diese Weise, werden alle Funktionen des Endoskopschafts einschließlich der im Endoskopkopf verbauten Funktion wie Optik, Beleuchtung, Stellantriebe, etc. unmittelbar am Anschlusseinsatz unter Umgehung des Handgriffs angeschlossen.

Gemäß der vorliegenden Erfindung hat der Handgriff eine Betätigungseinrichtung, beispielsweise ein Tastenfeld, zur Aktuierung einer (separaten) Betriebsstation, welche an das Endoskop über die vorstehend genannten weiteren Anschlüsse angeschlossen/anschließbar ist. Die Steuerungs-Verbindung zwischen Handgriff und Betriebsstation erfolgt vorzugsweise kabellos insbesondere per W-Lan, Blue-Tooth oder Infrarot-Signal, wobei natürlich aber auch eine Kabelverbindung vorgesehen sein kann.

Weiter vorzugsweise ist am Handgriff ein Sensor oder dergleichen Erfassungsschalter angebracht, der den Anschlusseinsatz erfasst, wenn dieser mit dem Handgriff gekoppelt ist, um daraufhin die Aktuierung der Betriebsstation freizuschalten. Zusätzlich oder alternativ kann an den weiteren Anschlüssen ein ähnlicher Sensor/Erfassungsschalter mit gleicher Funktion und Wirkung angeordnet sein.

Gemäß der Erfindung bildet das Gehäuse/Aufnahmegehäuse des Handgriffs eine fluiddichte Ummantelung sowohl des Tastenfelds wie auch der darin aufgenommenen Elektronik. Im Fall einer Kabelverbindung ist auch der Anschluss zwischen Kabel und Handgriff vorzugsweise fluiddicht ummantelt. Der Handgriff bildet somit eine fluiddichte Einheit, die separat zum Endoskopschaft auf einfache Weise, beispielsweise durch ein Tauchverfahren, gereinigt werden kann.

An dieser Stelle sei auf die vorteilhafte Ausbildung der Offenbarung hingewiesen, wonach im Wesentlichen alle Funktionen des Endoskops durch die Betriebsstation aktuiert werden. Hierzu zählen weiter vorzugsweise das Aktuieren der Optik und deren Beleuchtung, das Aktuieren des distalen Abwinklungsabschnitts zur kontrollierten Abkrümmung/Abwinklung des Endoskops in dessen distalem Längenabschnitt, das Aktuieren des Einpressens von Spüllösungen oder Medikamenten oder weiteren Fluiden und das Aktuieren des Absaugens von Flüssigkeiten/Fluiden. Diese Funktionen sind mittels des Bedienfelds vorzugsweise elektrisch steuerbar, wofür das Bedienfeld über eine Signalleitung (beispielsweise W-Lan, Blue-Tooth, Infrarot oder Kabel) mit der Betriebsstation gekoppelt ist, welche mit der Kommunikationseinrichtung verbunden ist.

### Figurenbeschreibung

Die vorliegende Erfindung wird nachfolgend anhand eines bevorzugten Ausführungsbeispiels unter Bezugnahme auf die begleitenden Figuren näher erläutert.
Fig. 1 zeigt einen Handgriff sowie einen Anschlusseinsatz eines Endoskopschafts gemäß einem bevorzugten Ausführungsbeispiel und
Fig. 2 zeigt den Handgriff und den Anschlusseinsatz gemäß Fig. 1 in gekoppeltem Zustand mit einem Touchscreen als Bedienfeld.

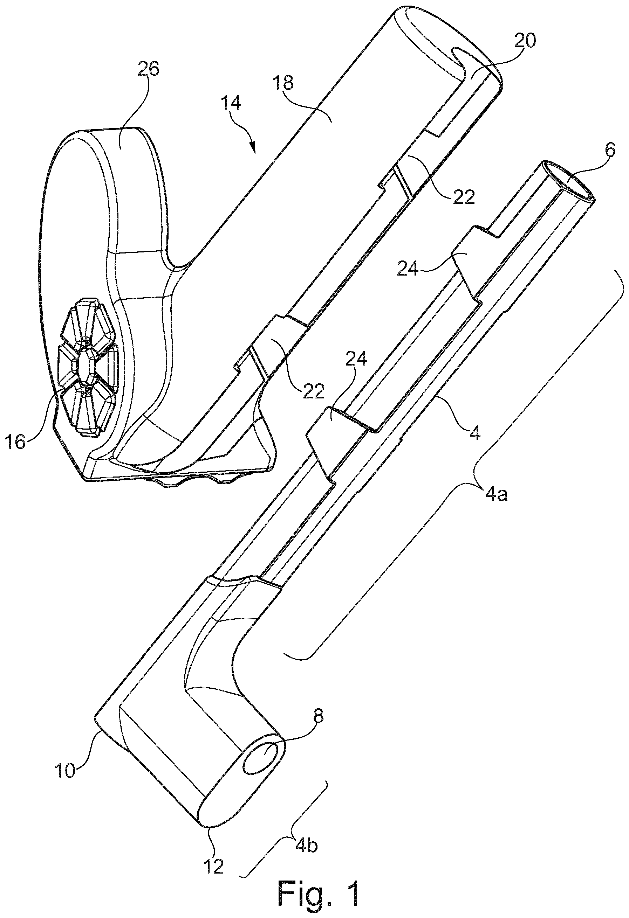

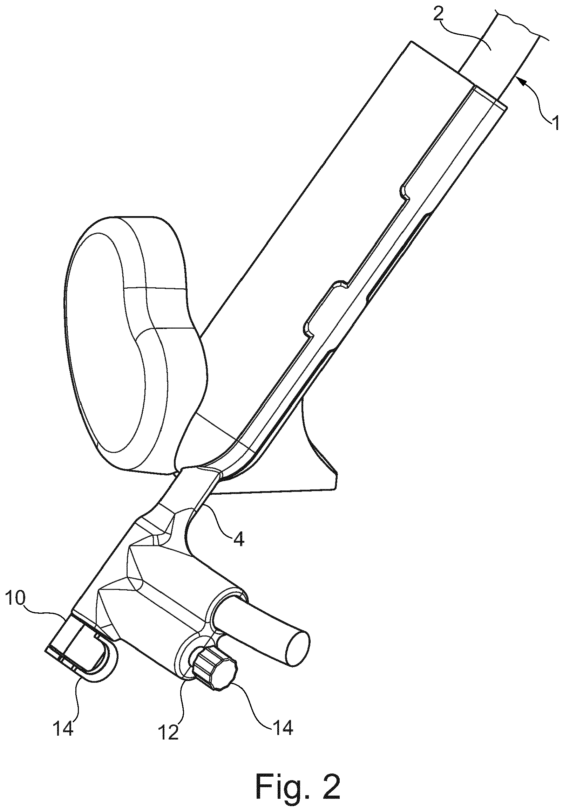

Gemäß der beiden Fig. 1 und 2 hat das Endoskop einen Endoskopschaft 1, an dessen nicht weiter dargestellten distalen Endbereich (Endoskopkopf) eine Anzahl von Funktionen verbaut ist. Diese Funktionen betreffen u. a. eine Optik, eine Beleuchtung, ggf. einen aktiv per Stellantrieb betätigbaren Abwinklungsabschnitt unmittelbar vor dem Endoskopkopf, und dergleichen. Insoweit entspricht der Endoskopschaft der vorliegenden Offenbarung dem bekannten Stand der Technik und braucht daher nicht näher beschrieben zu werden.

Im Unterschied zum allgemein bekannten Stand der Technik besteht der Endoskopschaft 1 der vorliegenden Offenbarung aus einem biegeflexiblen Schaftabschnitt 2, der dafür geeignet und dazu angepasst ist, in den Hohlraum (z.B. Darm oder Speiseröhre, etc.) eines Patienten unter passiver Nachverfolgung von Hohlraumkrümmungen/-windungen eingeführt zu werden und einem proximal daran anschließenden Anschlusseinsatz 4, der fest (oder auch lösbar) mit dem biegeflexiblen Schaftabschnitt 2 (koaxial) in axialer Verlängerung zu diesem verbunden ist. Vorzugsweise besteht der Anschlusseinsatz 4 aus einem Material, das steifer ist als der biegeflexible Schaftabschnitt 2.

Innerhalb des Endoskopschafts 1 umfassend den flexiblen Schaftabschnitt 2 sowie den Anschlusseinsatz 4 ist ein (zentraler) Arbeitskanal 6 sowie eine Anzahl von Versorgungs-/Funktionskanälen (nicht weiter dargestellt) ausgebildet. In den Versorgungs-/Funktionskanälen sind zum Teil (elektrisch oder lichtleitende) Leitungskabel verlegt, die an die vorstehend genannten Funktionen angeschlossen sind. Es können aber auch Hydraulikflüssigkeit-führende Versorgungs-/Funktionskanäle vorgesehen sein, etwa zur hydraulischen Betätigung des Abwinklungsabschnitts oder zur Kühlung/Reinigung einzelner Funktionen im Endoskopkopf.

Diese Versorgungs-/Funktionskanäle erstrecken sich ausgehend vom Endoskopkopf durch den biegeflexiblen Schaftabschnitt 2 und den sich proximal daran anschließenden Anschlusseinsatz 4 und enden in einer Anschlusseinrichtung 8 bzw. Funktionsanschlüssen (z.B. kombinierte Elektrik/Hydraulik-Steckdose) am proximalen Ende des Anschlusseinsatzes 4.

Der Arbeitskanal 6 erstreckt sich ebenfalls ausgehend vom Endoskopkopf durch den biegeflexiblen Schaftabschnitt 2 und den Anschlusseinsatz 4 und hat einen Durchmesser, der das gleitende Hindurchführen von minimalinvasiven chirurgischen Instrumenten mit standardisierten Außendurchmessern (aus dem allgemeinen Stand der Technik bekannt) erlaubt. Der Arbeitskanal 6 öffnet sich am Endoskopkopf in Richtung distal zur Umgebung, sodass ein chirurgisches Instrument (Zange, Schere, Pinzette, etc.) aus dem Endoskopschaft vorausgeschoben werden kann.

Am proximalen Ende des Arbeitskanals mündet dieser in wenigstens zwei separate (Arbeitskanal-) Anschlüsse 10, 12, welche im Anschlusseinsatz 4 ausgebildet sind.

Im Konkreten hat der Anschlusseinsatz 4 neben den vorstehend beschriebenen Funktionsanschlüssen 8 wenigstens zwei weitere Arbeitskanalanschlüsse 10, 12, die separat sowie unabhängig voneinander (auch gleichzeitig) benutzt werden können.

Vorzugsweise ist zumindest einer der Anzahl von Arbeitskanalanschlüssen 10 dazu vorgesehen (angepasst), dass über diesen ein chirurgisches Instrument in den Arbeitskanal 6 eingeführt wird. Demzufolge hat dieser Arbeitskanalanschluss 10 wenigstens einen Durchmesser, der für das Einführen des chirurgischen Instruments (mit Standardaußendurchmesser) ausreicht. Vorzugsweise entspricht der Durchmesser dieses Arbeitskanalanschlusses dem des Arbeitskanals oder ist (etwas) größer. Des Weiteren ist dieser Arbeitskanalanschluss 10 bezüglich des Arbeitskanals 6 vorzugsweise so ausgerichtet, dass das chirurgische Instrument bei dessen Einführen in den Arbeitskanal nicht abknickt (in einem stumpfen Winkel oder axial zum Arbeitskanal 6).

Weiter vorzugsweise ist zumindest einer der Anzahl von Arbeitskanalanschlüssen 12 dazu vorgesehen (angepasst), dass über diesen ein Fluid in den Arbeitskanal 6 eingeführt oder abgeführt wird. Beispielsweise hat dieser Anschluss 12 eine Vorrichtung, die ein fluiddichtes Anschließen einer (nicht gezeigten) Pumpe, Spritze oder dergleichen ermöglicht. Eine solche Vorrichtung kann ein einfacher Rohrstutzen sein, auf den z.B. ein Schlauch aufgeschoben ist/wird oder ein Bajonett-, Schraub- oder Luerverschluss und dergleichen.

Jeder der Arbeitskanalanschlüsse 10, 12 kann ferner mit einem Verschluss (Deckel, Kappe, Blindstopfen, etc.) 14 versehen sein, mittels dem bei Nichtnutzung des jeweiligen Arbeitskanalanschlusses 10, 12 dieser fluiddicht verschlossen werden kann.

Vorzugsweise sind der eine Arbeitskanalanschluss 12 für das Ein-/Abführen von Fluid 12 und der Funktionsanschluss 8 in einem (stumpfen) Winkel (maximal 90°) zum Arbeitskanal 6 innerhalb des Anschlusseinsatzes 4 ausgerichtet. Weiter vorzugsweise verlaufen der eine Arbeitskanalanschluss 12 für das Ein-/Abführen von Fluid 12 und der Funktionsanschluss 8 parallel zueinander (auf einer Winkelposition bezüglich des Arbeitskanals 6). Sie können aber auch in Umfangsrichtung des Arbeitskanals versetzt zueinander angeordnet sein.

Insoweit bildet der Anschlusseinsatz 4 in seinem äußeren Erscheinungsbild quasi einen (distalen) Rohrlauf (in Verlängerung zum biegeflexiblen Schaftabschnitt) 4a und einen (proximalen) Anschlussstutzen 4b bestehend aus den Arbeitskanalanschlüssen 10, 12 und den weiteren (Funktions-) Anschlüssen 8 für die eingangs genannten Endoskopfunktionen.

Der so aufgebaute Endoskopschaft 1 (bestehend aus dem Anschlusseinsatz 4, dem biegeflexible Schaftabschnitt 2, ggf. dem aktiv abwinkelbaren Abschnitt und dem daran sich anschließenden Endoskopkopf - in dieser Reihenfolge) beinhaltet alle Funktionen des Endoskops sowie deren Anschlüsse 8, 10, 12 für das Anschließen an eine (nicht dargestellte) separate Betriebseinheit/-station. Eine solche Station umfasst u.a. Einrichtungen zum Betreiben der Optik und der Beleuchtung (ggf. einschließlich deren Kühlung), Einrichtungen für das Betätigen des Abwinklungsabschnitts, Einrichtungen (Pumpen) für das Einpressen von Spüllösungen oder Medikamenten und/oder das Absaugen von Flüssigkeiten, etc..

Aus diesem Grund ist es bevorzugt, den so definierten Endoskopschaft 1 als Ein-Weg-Artikel zu konzipieren, um aufwändige Reinigungsarbeiten zu vermeiden. Hier hat sich gezeigt, dass sowohl die Funktionen wie auch deren Anschlüsse preisgünstig hergestellt werden können, sodass eine Ein-Weg-Bauweise nicht nur aus hygienischen Gründen vorteilhaft ist sondern sich auch als wirtschaftlich erweist.

Das Endoskop hat einen Handgriff 14, mittels dem das Endoskop/der Endoskopschaft 1 vorzugsweise gemäß vorstehender Definition in einer Endoskopie-Anwendung gehalten werden kann. Vorzugsweise sind im/am Handgriff 14 Betätigungselemente angeordnet, mittels denen die Funktionen des Endoskops aktuiert werden können.

Beispielsweise hat der Handgriff 14 ein Bedienfeld/Bedienerfeld 16 vorzugsweise in Form einer Tastatur, Joystick, Platte oder eines Touchscreen, die sich in ergonomisch günstiger Weise an einen Griffabschnitt 18 anschließt, derart, dass das Bedienfeld 16 mit den Fingern der zur Greifhand zweiten Hand betätigt werden kann. Optional kann alternativ oder zusätzlich zu dem Bedienfeld 16 ein (nicht weiter gezeigter) Lage-/G-Sensor/ Beschleunigungssensor bekannter Konstruktion integriert werden, um über z. B. eine Bewegung des Handgriffs die Endoskopspitze zu steuern.

Vorzugsweise ist der Griffabschnitt 18 als Halb- oder Drei-Viertelschale ausgebildet und definiert eine Art Klammer oder Manschette, die auf den Endoskopschaft 1 und vorzugsweise auf den Rohrlauf 4a des Anschlusseinsatzes 4 aufgesteckt werden kann. Hierfür hat der Griffabschnitt 18 beispielsweise eine Rohrform mit durchgehenden Längsschlitz 20, wobei im Längsschlitz 20 eine Anzahl von Zacken oder dergleichen Hinterschneidungen (Ausnehmungen) 22 ausgebildet sind. Die Rohrform ist gemäß diesem Ausführungsbeispiel im Querschnitt oval ausgebildet, um gut gehalten werden zu können und dem Greifer auch ein taktiles Gefühl für die Rotationslage des Endoskopschafts zu vermitteln. Es ist aber auch denkbar, den Griffabschnitt rund oder eckig auszubilden.

Der Anschlusseinsatz 4 hat im Bereich seines Rohrlaufs 4a ebenfalls eine Anzahl von Hinterschneidungen (Vorsprüngen) 24, die bei Einsetzen in den schalenförmigen Griffabschnitt 18 des Handgriffs 14 mit den dort ausgebildeten Hinterschneidungen 22 in Eingriff kommen und so die Relativposition des Handgriffs 14 zum Anschlusseinsatz 4 (und damit zum gesamten Endoskopschaft) festlegen.

Der Handgriff 14 bildet zumindest im Bereich des Bedienfeldes 16 ein Gehäuse/Aufnahmegehäuse 26, in dem elektrische/elektronische Bauteile zur Betätigung der separaten Betriebseinheit/-station untergebracht sind. Vorzugsweise betreffen diese elektrischen/elektronischen Bauteile eine kabellose Kommunikationseinrichtung sowie deren Energieversorgung. Als kabellose Kommunikationseinrichtung kommen beispielsweise W-Lan, Blue-Tooth oder Infrarot in Frage. Es ist aber auch denkbar, am Handgriff 14 eine Verkabelung anzuordnen, über die der Handgriff 14 (elektrisch) mit der Betriebseinheit/-station verbunden werden kann/ist.

Das Gehäuse/Aufnahmegehäuse 26 ist dabei fluiddicht oder aber mit einer fluiddichten Hülle (vorzugsweise nur im Bereich des Bedienfeldes 16) ummantelt, sodass dessen Oberfläche, beispielsweise durch einfaches Eintauchen in eine Reinigungsflüssigkeit, gesäubert werden kann.

Das Bedienfeld 16 in Fig. 1 weist in dieser Ausführungsform Bedienelemente/ Funktionstasten auf, welche kreisförmig / konzentrisch um eine mittige Funktionstaste angeordnet sind und normal gegenüber der Oberfläche des Aufnahmegehäuses 26 nach außen hin vorstehen / vorspringen. Dies ermöglicht dem Bediener unter anderem für eine eindeutige Haptik und eine gute Bedien-Zugänglichkeit der Funktionstasten. Alle Funktionstasten des Bedienfeldes 16 sind weiter mit einer (flexiblen) Membran/fluiddichten Oberfläche überzogen oder weisen eine fluiddichtes Material auf, welche radial nach außen in das Aufnahmegehäuse 26 übergehen bzw. fluiddicht angebunden sind. Die Membranen/Oberflächen der Funktionstasten sind ebenso untereinander/miteinander fluiddicht verbunden, wodurch letztlich die Funktionstasten und damit auch das Bedienfeld 16 nach außen fluiddicht verschlossen sind. Das Aufnahmegehäuse 26, welches die elektrischen Bauteile der Kommunikationseinrichtung beherbergt, kann beispielsweise als Spritzgussteil ausgeführt sein, um das Aufnahmegehäuse 26 ebenfalls nach außen fluiddicht zu verschließen, wodurch der Bereich der Elektrik/Elektronik fluiddicht verschlossen ist.

In Figur 2 ist eine weitere Ausführungsform des Bedienfeldes 16 mit Touchscreen 16 gezeigt. Die Oberfläche des Touchscreens 16 ist fluiddicht ausgelegt und eine Anbindung an das Aufnahmegehäuse 26 so gestaltet, beispielsweise über eine umlaufende Dichtung, dass dieser Anbindungsbereich nach außen fluiddicht verschlossen ist. Über eine entsprechende fluiddichte Ausführung des Aufnahmegehäuses 26 ist somit der Handgriff 14 wieder für eine Nassreinigung angepasst.

Das Aufnahmegehäuse 26 kann in einer Ausführungsform an einer Seite eine Öffnung aufweisen, um eine Zugänglichkeit zu den elektrischen Bauteilen zu ermöglichen. Über einen verschließbaren Deckel / Element / Bauteil mit umlaufender Dichtung lässt sich das Aufnahmegehäuse 26 wieder fluiddicht gestalten. Auch ist denkbar, den Touchscreen 16 abnehmbar/lösbar/ab- und ankoppelbar zu gestalten. In einer weiteren Ausführung kann um die elektronischen Bauteile und an das Bedienfeld 16 ein komplettes Aufnahmegehäuse 26 abschließend fluiddicht (an-)geformt werden.

Erfindungsgemäß ist der Handgriff 14 mit einer fluiddichten Hülle ummantelt, die eine Oberflächenbeschichtung durch Eintauchen in eine geeignete Versiegelungsflüssigkeit, welche bspw. temperaturabhängige Aggregatzustände aufweist oder chemisch aushärtet, ist.

Optional ist am Handgriff 14 im Bereich des Griffabschnitts 18 ein Sensor oder Schalter (nicht gezeigt) vorgesehen, der den Anschlusseinsatz 4 dann registriert, wenn dieser in den Handgriff 14 (dessen Griffabschnitt 18 mit Schalenform) eingesetzt ist. Der Schalter/Sensor ist mit der Elektronik im Handgriff 14 verbunden und schaltet diese nur dann frei, wenn der Handgriff 14 korrekt mit dem Endoskopschaft 1 mechanisch gekoppelt ist. So wird das Betätigen der Endoskopfunktionen bei nicht/falsch gekoppeltem Handgriff 14 verhindert. Weiter optional ist der Handgriff für Rechts- oder Linkshänder und für unterschiedliche Handgrößen konfigurierbar.

Gemäß der anliegenden Figuren ist das Bedienfeld 16 in Form einer Scheibe/Platte ausgebildet, die sich unter einem spitzen Winkel sowie seitlich zum Griffabschnitt 18 erstreckt. Auf diese Weise kann der Griffabschnitt 18 mit einer Hand (z.B. der rechten Hand) ergriffen und parallel dazu das Bedienfeld 16 mit der anderen Hand betätigt werden.

Schließlich ist aus den Figuren zu ersehen, dass bei in den Handgriff 14 (dessen Griffabschnitt 18) eingesetztem Anschlusseinsatz 4 die daran ausgebildeten Anschlüsse (Funktionsanschlüsse und Arbeitskanalanschlüsse/-zugänge) 8-12 aus dem Handgriff 14 (dessen Griffabschnitt 18) vorzugsweise proximal herausragen. Sie sind daher auch bei eingesetztem Zustand des Anschlusseinsatzes 4 von außen frei zugänglich. Es ist somit möglich, Anschlüsse nach Einsetzen des Anschlusseinsatzes 4 in den Handgriff 14 beliebig zu nutzen oder wieder zu verschließen.

## Patentansprüche

1. Handgriff (14) für ein Endoskop, mit einem Griffabschnitt (18) und einer Kommunikationseinrichtung,
wobei an dem Griffabschnitt (18) ein Aufnahmegehäuse (26), vorzugsweise stoffeinstückig, angeformt ist, in welchem elektrische und/oder elektronische Bauteile der Kommunikationseinrichtung untergebracht sind und an dessen Außenseite ein Bedienfeld (16), über das eine separate, an das Endoskop über Anschlüsse angeschlossene Betriebsstation aktuierbar ist, angeordnet ist, das mit der Kommunikationseinrichtung in elektrischer Verbindung steht,
wobei der Handgriff (14) mit einer fluiddichten Hülle ummantelt ist, sodass das Aufnahmegehäuse (26) und das Bedienfeld (16) nach außen fluiddicht verschlossen sind, sodass der Handgriff (14) für eine Nassreinigung, vorzugsweise durch ein Tauchverfahren, angepasst ist,
**dadurch gekennzeichnet, dass**
die fluiddichte Hülle eine Oberflächenbeschichtung durch Eintauchen in eine geeignete Versiegelungsflüssigkeit ist.

2. Handgriff (14) nach Anspruch 1, **dadurch gekennzeichnet, dass** zusätzlich zu dem Bedienfeld (16) ein Lagesensor zur handgriffsbewegungsabhängigen Steuerung des Endoskops innerhalb des Handgriffs (14) vorgesehen ist.

3. Endoskop mit einem Endoskopschaft (1), an dessen distalem Ende zumindest eine Optik sowie eine Beleuchtung vorgesehen sind, die über Funktions- und/oder Versorgungskanäle innerhalb des Endoskopschafts (1) dafür angepasst sind, an eine Betriebseinheit oder Betriebsstation angeschlossen zu sein und einem am distalen Ende des Endoskopschafts (1) sich öffnenden Arbeitskanal (6), der im Endoskopschaft (1) ausgeformt und dafür angepasst ist, chirurgische Instrumente der Minimalinvasivbauart längsverschieblich aufzunehmen, **gekennzeichnet durch** einen Handgriff (14) gemäß einem der Ansprüche 1 bis 2, der als ein zum Endoskopschaft (1) separates Bauteil vorgesehen ist.

4. Endoskop nach Anspruch 3, **dadurch gekennzeichnet, dass** der Endoskopschaft (1) zumindest einen biegeflexiblen Abschnitt (2) hat, der dazu geeignet und angepasst ist, bei einer Vorwärtsbewegung des Endoskops in einen Hohlraum eines Patienten unter passiver Nachverfolgung von Hohlraumwindungen zu folgen und der Endoskopschaft (1) einen proximalen vergleichsweise starren, daran anschließenden Anschlusseinsatz (4) hat, der mit dem biegeflexiblen Abschnitt (2) in axialer Verlängerung zu diesem verbunden ist und an dem Anschlüsse (8 bis 12) für die Funktions- und/oder Versorgungskanäle sowie des Arbeitskanals (6) ausgebildet oder angeordnet sind.

5. Endoskop nach Anspruch 4, **dadurch gekennzeichnet, dass** der Anschlusseinsatz (4) einen Rohrlauf (4a) hat, dessen eines Ende mit dem Arbeitskanal (6) innerhalb des mit dem Anschlusseinsatz (4) fest verbundenen biegeflexiblen Schaftabschnitts (2) fluidverbunden ist und an dessen anderem Ende ein Anschlussstutzen (4b) angeordnet ist, an dem die Anschlüsse (8 bis 12) für die Funktions- und/oder Versorgungskanäle sowie des Arbeitskanals (6) vorgesehen sind.

## Claims

1. Handle (14) for an endoscope, comprising a handle portion (18) and a communication device,
wherein an accommodation housing (26), in which electrical and/or electronic components of the communication device are accommodated, is preferably integrally formed on the handle portion (18), and on the outside of which a control panel (16) is arranged, via which a separate operating station connected to the endoscope via connections can be actuated, the control panel being electrically connected to the communication device,
wherein the handle (14) is surrounded by a fluid-tight sleeve, so that the accommodation housing (26) and the control panel (16) are closed fluid-tight to the outside so that the handle (14) is adapted for wet cleaning, preferably by a dipping method,
**characterized in that**
the fluid-tight sleeve is a surface coating by immersion in a suitable sealing liquid.

2. Handle (14) according to claim 1, **characterized in that** in addition to the control panel (16), a position sensor is provided for controlling the endoscope within the handle (14) as a function of handle movement.

3. Endoscope with an endoscope shaft (1), at the distal end of which at least one optics as well as an illumination are provided, which are adapted via functional and/or supply channels within the endoscope shaft (1) to be connected to an operating unit or an operating station, and with a working channel (6) which opens at the distal end of the endoscope shaft (1), and which is formed in the endoscope shaft (1) and adapted to receive surgical instruments of the minimally invasive type in a longitudinally displaceable manner, **characterized by** a handle (14) according to one of the claims 1 to 2, which is provided as a component separate from the endoscope shaft (1).

4. Endoscope according to claim 3, **characterized in that** the endoscope shaft (1) has at least one flexurally flexible section (2) which is suitable and adapted to follow a forward movement of the endoscope into a cavity of a patient with passive tracking of cavity windings, and the endoscope shaft (1) has an adjoining, proximal, comparatively rigid connection insert (4), which is connected to the flexurally flexible section (2) in axial extension thereto, and on which connections (8 to 12) for the functional and/or supply channels and the working channel (6) are formed or arranged.

5. Endoscope according to claim 4, **characterized in that** the connection insert (4) has a pipe rail (4a), one end of which is fluidly connected to the working channel (6) within the flexurally flexible shaft portion (2) fixedly connected to the connection insert (4), and at the other end of which a connection nozzle (4b) is arranged on which the connections (8 to 12) for the functional and/or supply channels and the working channel (6) are provided.

## Revendications

1. Poignée (14) pour un endoscope, avec une portion de poignée (18) et un dispositif de communication,
moyennant quoi, sur la portion de poignée (18), est moulé un boîtier de logement (26), de préférence d'une seule pièce, dans lequel sont logés des composants électriques et/ou électroniques du dispositif de communication et sur le côté extérieur duquel un panneau de commande (16), par l'intermédiaire duquel une station de commande raccordée à l'endoscope par l'intermédiaire de raccordements, peut être actionnée, qui est reliée électriquement avec le dispositif de communication, la poignée (14) étant entourée d'une gaine étanche aux fluides, de façon à ce que le boîtier de logement (26) et le panneau de commande (16) soient scellés vers l'extérieur de manière étanche aux fluides, de façon à ce que la poignée (14) soit adaptée à un nettoyage par immersion, de préférence par un procédé de trempage,
**caractérisée en ce que**
la gaine étanche aux fluides est un revêtement de surface par immersion dans un fluide de scellement approprié.

2. Poignée (14) selon la revendication 1, **caractérisée en ce que**, en plus du panneau de commande (16), un capteur de position est prévu à l'intérieur de la poignée pour la commande de l'endoscope en fonction des mouvements de la poignée (14).

3. Endoscope avec une tige d'endoscope (1), à l'extrémité distale de laquelle au moins une optique ainsi qu'un éclairage sont prévus, qui sont conçus, par l'intermédiaire de canaux de fonctionnement et/ou d'alimentation à l'intérieur de la tige de l'endoscope (1), pour être raccordés à une unité de commande ou une station de commande et pour loger un canal de travail (6) s'ouvrant à l'extrémité distale de la tige d'endoscope (1), qui est formé dans la tige de l'endoscope (1) et est conçu pour loger de manière coulissante longitudinalement des instruments chirurgicaux minimalement invasifs, **caractérisé par** une poignée (14) selon l'une des revendications 1 à 2, qui est conçue comme un composant séparé de la tige d'endoscope (1).

4. Endoscope selon la revendication 3, **caractérisé en ce que** la tige d'endoscope (1) comprend au moins une portion flexible (2) qui est conçu et adapté pour suivre, lors d'un mouvement vers l'avant de l'endoscope dans une cavité d'un patient, par poursuite passive de spires de la cavité et la tige d'endoscope (1) comprend un insert de raccordement (4) proximal relativement rigide, qui y est raccordé, qui est relié avec la portion flexible (2) dans le prolongement axial de celle-ci et est réalisé ou disposé au niveau des raccordements (8 à 12) pour les canaux de fonctionnement et/ou d'alimentation ainsi que du canal de travail (6).

5. Endoscope selon la revendication 4, **caractérisé en ce que** l'insert de raccordement (4) comprend un tube (4a) dont une extrémité est reliée de manière fluidique avec le canal de travail (6) à l'intérieur de la portion de tige flexible (2) relié fermement avec l'insert de raccordement (4) et à l'autre extrémité duquel est disposé un manchon de raccordement (4b) au niveau duquel les raccordements (8 à 12) pour les canaux de fonctionnement et/ou d'alimentation ainsi que du canal de travail (6) sont prévus.
